# EUROPÄISCHE PATENTSCHRIFT

(11) **EP 1 715 786 B1**
(45) Veröffentlichungstag und Bekanntmachung des Hinweises auf die Patenterteilung: **08.04.2015**
(21) Anmeldenummer: 05707440.3
(22) Anmeldetag: 16.02.2005
(51) Int. Cl.: A61B 1/00, G02B 6/38, A61B 1/07, G02B 23/24

(54) **VERFAHREN ZUM FIXIEREN VON GLASFASERN IN EINEM ENDOSKOP**
METHOD FOR FIXING OPTICAL FIBRES IN AN ENDOSCOPE
PROCEDE DE FIXATION DE FIBRES DE VERRE DANS UN ENDOSCOPE

(30) Priorität: 16.02.2004 DE 102004008458
(43) Veröffentlichungstag der Anmeldung: 02.11.2006
(73) Patentinhaber: Karl Storz GmbH & Co. KG, 78532 Tuttlingen (DE)
(72) Erfinder: HÖFIG, Siegfried, 78570 Mühlheim (DE)
(74) Vertreter: Weller, Wolfgang
(86) Internationale Anmeldenummer: PCT/EP2005/001577
(87) Internationale Veröffentlichungsnummer: WO 2005/077254

(56) Entgegenhaltungen:
- EP-A- 0 072 193
- EP-A- 0 072 194
- DE-A1- 10 164 582
- US-A- 4 350 150
- US-A- 6 085 011

## Beschreibung

Die vorliegende Offenbarung betrifft ein Verfahren zum Fixieren von Glasfasern in einem Endoskop, an einem proximalen Lichtanschluss des Endoskops, das folgende Schritte aufweist, nämlich
a) Einführen eines proximalen Endbereichs der Glasfasern in eine Hülse;
b) Einführen der Hülse in einen am Endoskop angeordneten Stutzen des Lichtanschlusses; und
c) Fixieren des proximalen Endbereichs der Glasfasern in der Hülse.

Das beschriebene Verfahren wurde bisher von der Anmelderin bei der Montage von Endoskopen verwendet und eine entsprechende vorgehensweise ist aus US4350150 bekannt.

Der proximale Endbereich der Glasfasern in Form eines Bündels wird dabei fest sitzend in die Hülse eingebracht. Es ist möglich, die Glasfasern vor oder nach dem Einführen der Hülse in den Stutzen in dieser zu fixieren. Die Hülse wird in den Stutzen des Lichtanschlusses eingeschraubt. Das proximal überstehende Ende des fixierten Glasfaserbündels wird abgelängt und bearbeitet, meist geschliffen und poliert.

Wird die Hülse mit dem darin fixierten proximalen Endbereich der Glasfasern in den Stutzen des Lichtanschlusses eingeschraubt, kommt es zu einem Verdrillen der Glasfasern, und zwar unabhängig davon, ob das Glasfaserbündel am gegenüberliegenden, also dem distalen Ende, schon im Endoskop fixiert ist oder nicht. Schon durch dieses Verdrillen der Glasfasern kann es zum Brechen einzelner Fasern kommen, was zu einer Reduzierung der Menge des geleiteten Lichtes führt.

Weiterhin setzt ein solches Verdrillen die Glasfasern unter Zugspannung, was zusätzlich zu einer Beschädigung der Glasfasern führen kann. Dieses wird noch dadurch verstärkt, dass Endoskope im medizinischen Bereich regelmäßig autoklaviert werden müssen, wobei sie kurzzeitig auf Temperaturen von über 150 °C aufgeheizt und danach wieder schnell abgekühlt werden. Hierbei kommt es zu unterschiedlichen Ausdehnungen von Teilen des Endoskops, die aus verschiedenen Materialien hergestellt sind. Dies kann die durch das Verdrillen erzeugten Spannungen noch verstärken und zu weiteren Beschädigungen der Glasfasern führen, z.B. wenn der Schaft des Endoskops sich stärker ausdehnt als die darin enthaltenen Glasfasern.

Auch ein nur loses Aufnehmen der Glasfasern in der Hülse ist nicht unproblematisch, da es beim Einschrauben zu einem Schleifen der Glasfasern an der Innenseite der Hülse kommt, was zu Abrieb und wiederum zu einer Beschädigung der hauchdünnen Fasern führen kann.

Es ist daher Aufgabe der vorliegenden Erfindung, das oben genannte Verfahren so weiterzubilden, dass eine möglichst effektive Lichteinspeisung und Lichtleitung erfolgt.

Erfindungsgemäß wird die Aufgabe durch ein Einführen der Hülse in den Stutzen durch eine lineare Bewegung in Richtung ihrer Längsachse gelöst.

Das Einführen durch eine lineare Bewegung kann hierbei durch ein einfaches Einstecken der Hülse in den Stutzen erfolgen. Es ist allerdings auch möglich, dass am Stutzen z.B. ein drehbarer Bajonettverschluss angebracht ist, der mit einem an der Hülse angebrachten Stift zusammenwirkt, so dass durch eine Drehung des Bajonettverschlusses die Hülse linear in den Stutzen eingeschoben wird.

All den oben genannten Ausführungsformen ist gemeinsam, dass die Hülse eine lineare Bewegung in Richtung ihrer Längsachse ausführt.

Es hat sich gezeigt, dass es beim Einführen der Hülse in den Stutzen durch eine lineare Bewegung nicht mehr zum Verdrillen der Glasfasern kommt. Hierdurch kann ein Brechen einzelner Fasern vermieden werden.

Es hat sich weiterhin gezeigt, dass durch das Einführen der Hülse durch eine lineare Bewegung die in der Hülse fixierten Glasfasern zugentlastet werden. Die im Inneren des Endoskops verlaufenden Glasfasern werden dabei etwas in das Endoskop eingeschoben. Hierdurch können während des Gebrauchs auftretende Dehnungsspannungen durch eine thermische Wechselbelastung zumindest abgemildert, wenn nicht sogar vollständig ausgeglichen werden.

Somit können durch die oben genannte Maßnahme Glasfasern ohne Beschädigung und unter deutlich geringerer Belastung im Lichtanschluss eines Endoskops fixiert werden.

Gemäß der Erfindung weist das Verfahren zwei weitere Schritte auf, nämlich
d) Abtrennen eines Endstücks der Hülse samt den darin enthaltenen Glasfasern; und
e) Bearbeiten einer Endfläche der Hülse und der darin enthaltenen Glasfasern.

Durch diese Maßnahme wird auf einfache Weise eine plane Endfläche an der Hülse und den Glasfasern geschaffen.

Nach dem Bearbeiten der Endfläche z.B. durch Schleifen und Polieren kann ein Mittel zum Einkoppeln von Licht plan auf die Hülse und die Glasfasern aufgesetzt werden, was zu einem Einkoppeln des Lichtes mit hoher Lichtausbeute führt.

Weiterhin können durch diese Maßnahme Hülsen verwendet werden, die länger als der Stutzen sind, was das Einführen der Hülse erleichtert. Durch das Abtrennen eines Teils der Hülse wird sichergestellt, dass die Hülse und die Glasfasern in dem fertigen Lichtanschluss plan abschließen.

In einer weiteren Ausgestaltung der Erfindung erfolgt das Einführen der Hülse in zwei Stufen, nämlich Einführen in eine erste Stellung, in der die Hülse in den Stutzen eingepresst ist, und dann Überführen in eine zweite, weiter eingeschobene Stellung, in der die Hülse axial beweglich im Stutzen verriegelt ist. Es ist dabei besonders vorteilhaft, das Überführen der Hülse in die zweite Stellung im Anschluss an Schritt e) durchzuführen.

Durch die oben genannte Maßnahme ist es möglich, die Hülse zuerst in dem Stutzen zu fixieren, so dass an der Hülse bzw. an dem darin eingeführten proximalen Endbereich der Glasfasern Manipulationen durchgeführt werden können. Insbesondere können dadurch die Glasfasern und gegebenenfalls ein Teil der Hülse abgelängt werden und die Endfläche geschliffen und poliert werden, während diese Endfläche noch in einem gewissen Maße über den Stutzen hinaussteht.

Danach kann die Hülse in eine zweite Stellung überführt werden, in der sie axial beweglich im Stutzen verriegelt ist. Diese axiale Beweglichkeit erlaubt es, z.B. Fertigungstoleranzen, wie sie beim Ablängen der Glasfasern bzw. beim Schleifen und Polieren der Endfläche auftreten können, auszugleichen und ermöglicht es somit, dass ein Mittel zum Einkoppeln von Licht in die Glasfasern plan an die Endfläche angelegt werden kann.

In einer weiteren Ausgestaltung der Erfindung ist ein federndes Element vorgesehen, das mit der Hülse derart zusammenwirkt, dass diese axial gefedert ist.

Dieses federnde Element hat den Vorteil, dass dadurch die Hülse mit den darin fixierten Glasfasern gegen ein Mittel zum Einkoppeln von Licht in die Glasfasern gedrückt werden kann, und dadurch ein möglichst genaues und planes Anliegen der Endfläche gegen dieses Mittel sichergestellt wird.

Das Vorsehen dieses federnden Elements hat außerdem den Vorteil, dass damit während des Gebrauchs des Endoskops z.B. durch thermische Ausdehnung auftretende Spannungen ausgeglichen werden können. Somit werden wiederum die auf die Glasfasern ausgeübten Belastungen reduziert.

In einer weiteren Ausgestaltung der oben genannten Maßnahme ist das federnde Element als O-Ring ausgebildet.

Ein O-Ring hat sich als eine besonders einfache und preisgünstige Ausführungsform des federnden Elements erwiesen.

Ein O-Ring hat weiterhin den Vorteil, dass er, außer dass er als federndes Element wirkt, gleichzeitig noch als Dichtung zwischen der Hülse und dem Stutzen wirkt. Dadurch kann z.B. ein Eindringen von Dampf während des Autoklavierens vermieden werden.

Gemäß der Erfindung weist das Verfahren einen weiteren Schritt auf, nämlich
f) Anbringen eines Faserkegels, derart, dass dieser an der Endfläche anliegt.

Das Bündel der zur Lichtleitung im Endoskop verwendeten Glasfasern weist auf Grund dessen, dass der Schaft des Endoskops normalerweise relativ dünn ist, im Allgemeinen einen sehr kleinen Durchmesser auf. Dies macht es schwierig, eine ausreichende Lichtmenge direkt in die Glasfasern einzukoppeln.

Ein Faserkegel dient hierbei dazu, die eingekoppelte Menge an Licht deutlich zu erhöhen.

Zusätzlich zu der oben genannten Maßnahme wird ein optisch leitfähiges Medium zwischen der Endfläche und dem Faserkegel eingebracht.

Es hat sich gezeigt, dass trotz hoher Genauigkeit bei der Fertigung zwischen der Endfläche und dem Faserkegel ein Spalt verbleibt. Dieser Spalt reduziert die Effektivität der Übertragung des Lichts von dem Faserkegel auf die Endfläche und damit die Effektivität des Einkoppelns von Licht in die Glasfasern. Es hat sich nun gezeigt, dass durch das Einbringen eines optisch leitfähigen Mediums zwischen der Endfläche und dem Faserkegel die Effektivität der Übertragung des Lichts von dem Faserkegel in die Glasfasern deutlich erhöht werden kann.

Durch diese Maßnahme ist es außerdem möglich, in Hülsen fixierte Glasfasern auf Vorrat vorzufertigen.

In einer weiteren Ausgestaltung der Erfindung erfolgt ein Fixieren des proximalen Endbereichs der Glasfasern in der Hülse durch Verkleben.

Verkleben hat sich als eine einfache, sichere und schnelle Technik erwiesen, die Glasfasern in der Hülse zu fixieren.

Es versteht sich, dass die vorstehend genannten und die nachstehend noch zu erläuternden Merkmale nicht nur in der jeweils angegebenen Kombination, sondern auch in anderen Kombinationen oder in Alleinstellung anwendbar sind, ohne den Rahmen der vorliegenden Erfindung zu verlassen.

Ein Ausführungsbeispiel der Erfindung ist in der Zeichnung dargestellt und wird in der nachfolgenden Beschreibung näher erläutert. Es zeigen:
- Fig. 1: einen Längsschnitt durch ein Endoskop in einem ersten Montagezustand, bei dem die Hülse noch nicht in den Stutzen einführt ist;
- Fig. 2: einen Abschnitt des Endoskops von Fig. 1, wobei die Hülse in den Stutzen des Endoskops eingeführt ist;
- Fig. 3: den Abschnitt von Fig. 2, wobei die Glasfasern in der Hülse fixiert sind;
- Fig. 4: den Abschnitt von Fig. 3, wobei die Glasfasern abgelängt sind;
- Fig. 5: den Abschnitt von Fig. 4, wobei die Hülse in eine zweite Stellung eingebracht ist; und
- Fig. 6: den Abschnitt von Fig. 5, wobei ein Faserkegel an die Hülse anliegend angebracht ist.

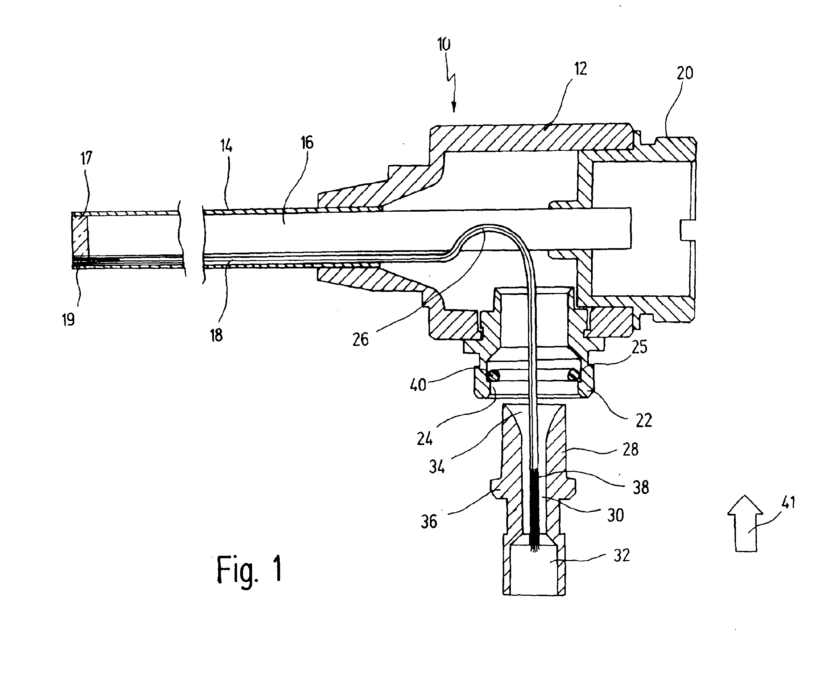

In Fig. 1 ist ein erfindungsgemäßes Endoskop in seiner Gesamtheit mit der Bezugsziffer 10 bezeichnet.

Das Endoskop 10 weist einen Kopf 12 auf, an den sich distal ein Schaft 14 anschließt. Der Schaft 14 ist hier nur abschnittsweise dargestellt.

In dem Schaft 14 verläuft ein Optikrohr 16. Dieses Optikrohr 16 dient zur Aufnahme des optischen Systems, wobei in dem Optikrohr 16 eine Vielzahl von hier nicht dargestellten Stablinsen aufgenommen sind. Das Optikrohr 16 wird am distalen Ende des Schafts 14 von einem Fenster 17 hermetisch abgeschlossen.

Parallel zu dem Optikrohr 16 verlaufen im Schaft 14 Glasfasern 18, die hier zu einem Bündel zusammengefasst sind. Die Glasfasern 18 sind mittels einer Klebstelle 19 am distalen Ende des Schafts 14 fixiert.

Proximalseitig des Kopfes 12 schließt sich eine Buchse 20 an, die dazu dient, ein Mittel zur Bilddarstellung, wie z.B. ein Okular oder eine Videokamera, mit dem Optikrohr 16 zu verbinden.

Am Kopf 12 ist weiterhin ein Stutzen 22 angeordnet, der Teil eines Lichtanschlusses des Endoskops 10 bildet.

Die Längsachse des Stutzens 22 verläuft in etwa im rechten Winkel zum Optikrohr 16, so dass der Lichtanschluss den Anschluss des Mittels zur Bilddarstellung an der Buchse 20 nicht beeinträchtigt.

An seiner Innenseite weist der Stutzen 22 in dessen äußerem Endbereich einen Ringflansch 24 auf, an den sich in Richtung des Kopfes 12 des Endoskops 10 eine Hinterschneidung 25 anschließt.

Die Glasfasern 18 reichen durch den Stutzen 22 aus dem Kopf 12 des Endoskops 10 heraus. Die Glasfasern 18 sind dabei zwischen dem Schaft 14 und dem Stutzen 22, also im Kopf 12, in einem Bogen 26 gelegt.

In Fig. 1 ist eine Hülse 28 dargestellt, die in den Stutzen 22 eingeschoben werden soll.

Die Hülse 28 weist in Längsrichtung eine durchgehende Bohrung 30 auf, die sich endoskopfern in eine erste Aufweitung 32 aufweitet und die sich endoskopnah in eine zweite, trichterförmige Aufweitung 34 aufweitet.

An ihrer Außenseite weist die Hülse 28 einen Ringflansch 36 auf.

Ein proximaler Endbereich 38 der Glasfasern 18 ist in die Bohrung 30 der Hülse 28 eingeführt. Die Hülse 28 wurde dabei über das seitlich vorstehende, proximale Ende der Glasfasern geschoben. Die endoskopnahe trichterförmige Aufweitung 34 erleichtert dabei das Einführen des proximalen Endbereichs 38 der Glasfasern 18 in die Bohrung 30 der Hülse 28.

In der Hinterschneidung 25 des Stutzens 22 ist ein O-Ring 40 aus elastischem Material angeordnet.

Die Hülse 28 wird nun durch eine lineare Bewegung in Richtung ihrer Längsachse, die durch den Pfeil 41 angedeutet ist, in den Stutzen 18 eingeführt.

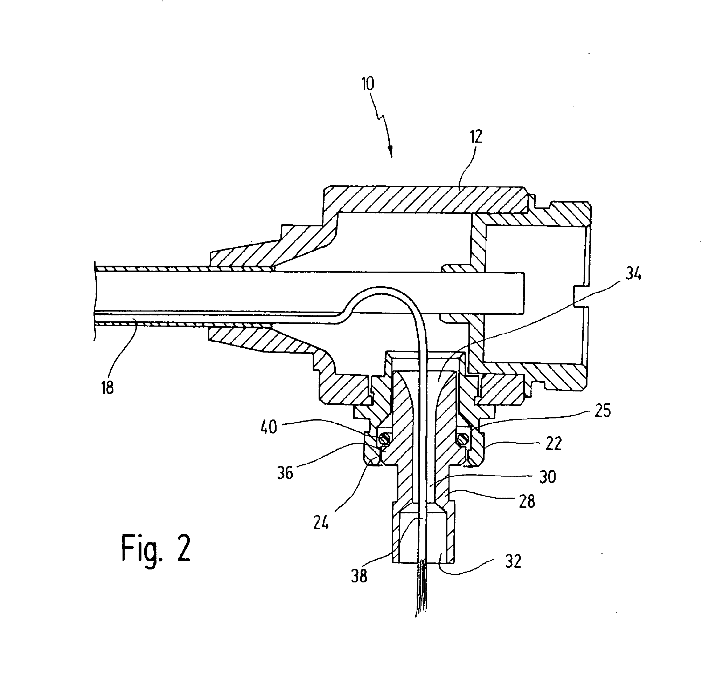

In Fig. 2 ist ein Abschnitt des Endoskops 10 nach dem in Fig. 1 durch den Pfeil 41 angedeuteten Einführen der Hülse 28 dargestellt.

Die Hülse 28 ist so weit in den Stutzen 22 eingeschoben, dass der Ringflansch 36 der Hülse 28 gegen den Ringflansch 24 des Stutzens 22 zu liegen kommt.

Der Ringflansch 36 der Hülse 28 wirkt mit dem Ringflansch 24 des Stutzens 22 derart zusammen, dass die Hülse 28 in der dargestellten Position unter Reibschluss oder Presssitz fixiert ist.

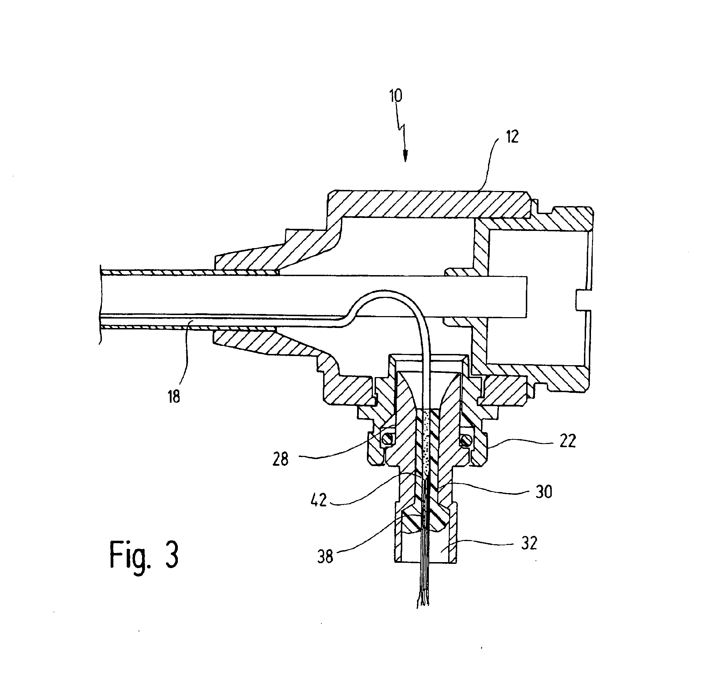

In Fig. 3 ist ein nächster Montageschritt dargestellt.

In diesem Schritt wurde der proximale Endbereich 38 der Glasfasern 18 in der Bohrung 30 der Hülse 28 fixiert.

Dieses erfolgte durch Einbringen einer Klebstoffmasse 42 in die Bohrung 30. Das Einbringen der Klebstoffmasse 42 in die Bohrung 30 wurde hierbei durch die endoskopferne Aufweitung 32 erleichtert. Die Klebstoffmasse 42 durchdringt auch das Bündel der Glasfasern 18.

Die Klebstoffmasse 42 wird ausgehärtet, was zu einer dauerhaften und einer dichten Verbindung zwischen dem proximalen Endbereich 38 der Glasfasern 18 und der Hülse 28 sowie der Glasfasern 18 untereinander führt.

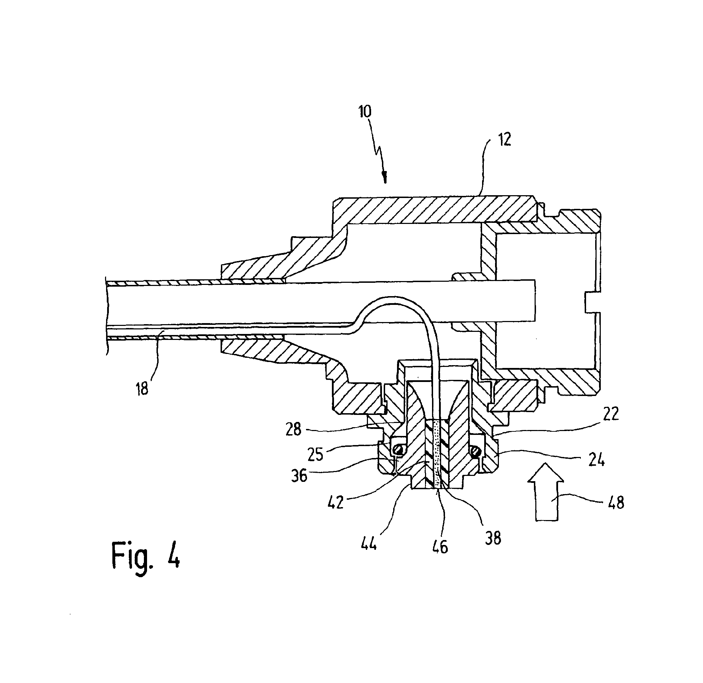

In Fig. 4 ist der Abschnitt des Endoskops 10 von Fig. 3 dargestellt, wobei die Hülse 28 mit dem darin fixierten proximalen Endbereich 38 der Glasfasern 18 abgelängt ist.

Das Ablängen erfolgt hierbei so, dass ein Abschnitt 44 der Hülse 28 und der darin enthaltenen Glasfasern 18 über den Stutzen 22 überstehen bleibt.

Durch das Ablängen wurde eine Endfläche 46 am proximalen Endbereich 38 der Glasfasern 18 bzw. der Hülse 28 gebildet. Diese Endfläche 46 wird noch bearbeitet, nämlich abgeschliffen und poliert, um ein möglichst effektives Einkoppeln von Licht in die Glasfasern 18 zu gewährleisten.

Während des Ablängens, des Schleifens und des Polierens ist die Hülse 28 durch das Einpressen in den Stutzen 22, also den Druck des Ringflansches 36 der Hülse 28 gegen den Ringflansch 24 des Stutzens 22 fest im Stutzen 22 fixiert. Hierdurch können die Manipulationen an der Hülse 28 bzw. den Glasfasern 18 sicher durchgeführt werden.

In einem nächsten Schritt wird nun die Hülse 28 mit dem darin enthaltenen proximalen Endbereich 38 der Glasfasern 18 in Richtung des Pfeils 48 weiter in den Stutzen 22 hineingeschoben. Hierdurch wird der Ringflansch 36 der Hülse 28 in die Hinterschneidung 25 des Stutzens 22 hinein bewegt. Auch dieses Hineinschieben erfolgt durch eine lineare Bewegung, wie dies durch den Pfeil 48 angedeutet ist.

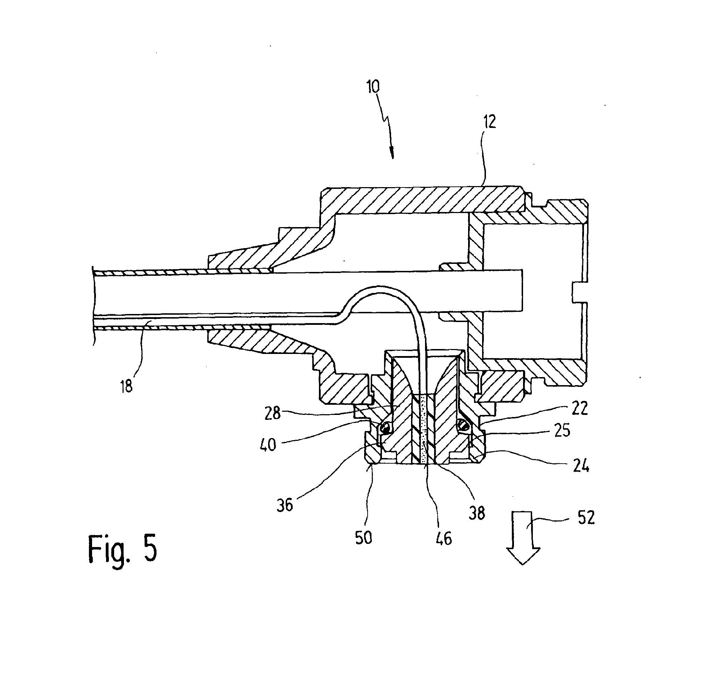

In Fig. 5 ist ersichtlich, dass der Ringflansch 36 in die Hinterschneidung 25 des Stutzens 22 eingerückt ist.

Die Endfläche 46 der Hülse 28, bzw. des proximalen Endbereichs 38 der Glasfasern 18, befindet sich jetzt in etwa auf gleicher Höhe wie die äußere ringförmige Stirnfläche 50 des Stutzens 22.

Der O-Ring 40 wird durch den Ringflansch 36 der Hülse 28 gegen den Rand der Hinterschneidung 25 des Stutzens 22 gedrückt und zusammengepresst. Der so zusammengepresste O-Ring 40 wirkt jetzt auf den Ringflansch 36, und damit auf die Hülse 28, einen Druck in Richtung des Pfeils 52 aus.

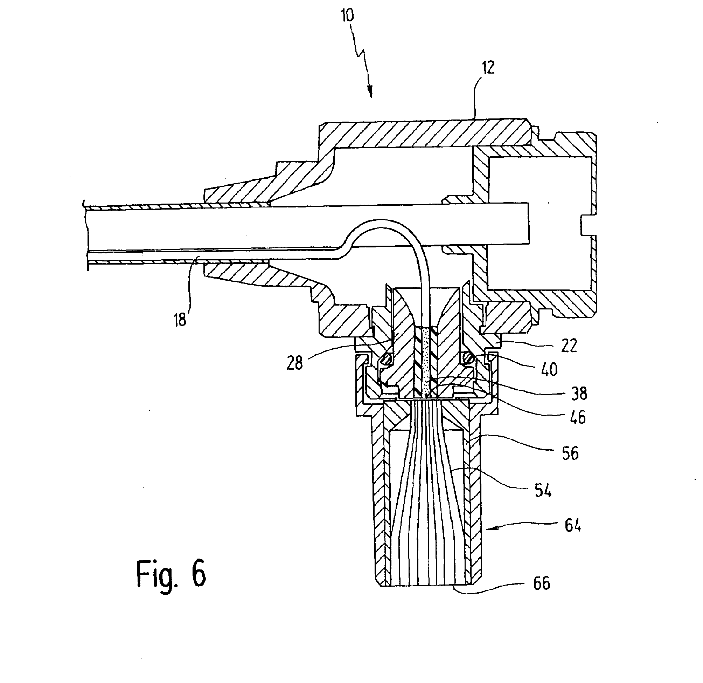

In Fig. 6 ist der Abschnitt des Endoskops 10 von Fig. 5 dargestellt, wobei an der Endfläche 46 der Hülse 28 bzw. des proximalen Endbereichs 38 der Glasfasern 18 ein Faserkegel 54 angeordnet ist.

Der Faserkegel 54 ist in einer Kegelhülse 56 aufgenommen, die auf die Außenseite des Stutzens 22 aufgeschraubt ist. Ein flüssiger Kitt wird in die Kegelhülse 56 eingebracht, der auch in die Freiräume zwischen Hülse 28 und Stutzen 22 eindringt. Der flüssige Kitt wird ausgehärtet, um einen dichten Abschluss zu schaffen.

Der Stutzen 22, die Hülse 28, der proximale Endbereich 38 der Glasfasern 18, der Faserkegel 54 und die Kegelhülse 56 bilden zusammen einen Lichtanschluss 64 des Endoskops 10.

Der O-Ring 40 drückt, wie dies in Fig. 5 durch den Pfeil 52 dargestellt ist, die Hülse 28 gegen den Faserkegel 54 und stellt somit ein möglichst planes Anliegen der Endfläche 46 an dem Faserkegel 54 sicher.

Um die Effizienz der Übertragung von Licht zwischen dem Faserkegel 54 und den Glasfasern 18 zu erhöhen, wird zwischen dem Faserkegel 54 und der Endfläche 46 ein optisch leitfähiges Medium eingebracht werden.

Über den Lichtanschluss 64 wird das Endoskop 10 mit einer externen Lichtquelle verbunden, die über die gesamte Breite der Kegelschnittfläche 66 Licht in den Faserkegel 54 einkoppelt. Dieses Licht wird längs des Faserkegels 54 geleitet, fokussiert und an der Endfläche 46 über einen kleineren Durchmesser in die Glasfasern 18 eingekoppelt.

## Patentansprüche

1. Verfahren zum Fixieren von Glasfasern (18) in einem Endoskop (10), an einem proximalen Lichtanschluss (64) des Endoskops (10), das folgende Schritte aufweist, nämlich
a) Einführen eines proximalen Endbereichs (38) der Glasfasern (18) in eine Hülse (28);
b) Einführen der Hülse (28) in einen am Endoskop (10) angeordneten Stutzen (22) des Lichtanschlusses (64);
c) Fixieren des proximalen Endbereichs (38) der Glasfasern (18) in der Hülse (28);
d) Abtrennen eines Endstücks der Hülse (28) samt den darin enthaltenen Glasfasern (18);
e) Bearbeiten einer Endfläche (46) der Hülse (28) und der darin enthaltenen Glasfasern (18); und
f) Anbringen eines Faserkegels (54) derart, dass dieser an der Endfläche anliegt,
wobei ein optisch leitfähiges Medium zwischen der Endfläche (46) und dem Faserkegel (54) eingebracht wird, und wobei ein Einführen der Hülse (28) in den Stutzen (22) durch eine lineare Bewegung in Richtung ihrer Längsachse erfolgt.

2. Verfahren nach Anspruch 1, gekennzeichnet durch ein Einführen der Hülse (28) in zwei Stufen, nämlich Einführen in eine erste Stellung, in der die Hülse (28) in den Stutzen (22) eingepresst ist und dann Überführen in eine zweite, weiter eingeschobene Stellung, in der die Hülse (28) axial beweglich im Stutzen (22) verriegelt ist.

3. Verfahren nach Anspruch 2, gekennzeichnet durch ein Überführen der Hülse (28) in die zweite Stellung im Anschluss an Schritt e).

4. Verfahren nach einem der Ansprüche 1 bis 3, gekennzeichnet durch ein Vorsehen eines federnden Elements, das mit der Hülse (28) derart zusammenwirkt, dass diese axial gefedert ist.

5. Verfahren nach einem der Ansprüche 1 bis 4, gekennzeichnet durch ein Fixieren des proximalen Endbereichs (38) der Glasfasern (18) in der Hülse (28) durch Verkleben.

6. Endoskop, mit einem proximalen Lichtanschluss (64), wobei der Lichtanschluss (64) eine Hülse (28) aufweist, in der ein proximaler Endbereich (38) von im Endoskop (10) aufgenommenen Glasfasern (18) zur Lichtleitung fixiert ist, und wobei die Hülse (28) in einem Stutzen (22) des Lichtanschlusses (64) angeordnet ist, wobei die Hülse (28) derart ausgebildet ist, dass sie durch eine,lineare Bewegung in Richtung ihrer Längsachse in den Stutzen (22) einführbar ist, wobei an einer Endfläche (46) der Hülse (28) und der darin enthaltenen Glasfasern (18) anliegend ein Faserkegel (54) angeordnet ist, und wobei zwischen der Endfläche (46) und dem Faserkegel (54) ein optisch leitfähiges Medium eingebracht ist.

7. Endoskop nach Anspruch 6, **dadurch gekennzeichnet, dass** die Hülse (28) nach dem Einführen im Stutzen (22) axial beweglich ist.

8. Endoskop nach Anspruch 7, **dadurch gekennzeichnet, dass** die Hülse (28) mit einem federnden Element derart zusammenwirkt, dass diese axial gefedert ist.

9. Endoskop nach Anspruch 8, **dadurch gekennzeichnet, dass** das federnde Element als O-Ring (40) ausgebildet ist.

10. Endoskop nach einem der Ansprüche 6 bis 9, **dadurch gekennzeichnet, dass** die Hülse (28) im Stutzen (22) verriegelt ist.

11. Endoskop nach einem der Ansprüche 6 bis 10, **dadurch gekennzeichnet, dass** der proximale Endbereich (38) der Glasfasern (18) durch Verkleben in der Hülse (28) fixiert ist.

## Claims

1. Method for fixing glass fibers (18) in an endoscope (10) at a proximal light connection (64) of the endoscope (10), comprising the following steps, namely:
a) introducing a proximal end section (38) of the glass fibers (18) into a sleeve (28);
b) introducing the sleeve (28) into a connecting piece (22) of the light connection (64) arranged on the endoscope (10);
c) fixing the proximal end section (38) of the glass fibers (18) in the sleeve (28);
d) disconnecting an end piece of the sleeve (28) together with the glass fibers (18) contained therein;
e) processing an end surface (46) of the sleeve (28) and the glass fibers (18) contained therein; and
f) fitting a fiber cone (54) in such a way, that the latter bears against the end surface,
wherein an optical conductive medium is introduced between the end surface (46) and the fiber cone (54), and wherein the introduction of the sleeve (28) into the connecting piece (22) is performed by a linear movement along its longitudinal axis thereof.

2. Method of claim 1 **characterized by** introducing the sleeve (28) in two steps, namely introducing it in a first position, in which the sleeve (28) is pressed into the connecting piece (22), and then transferring it into a second position, pushed further in, in which the sleeve (28) is locked within the connecting piece (22) but is axially movable therein.

3. Method of claim 1, **characterized by** transferring the sleeve (28) into the second position follows upon step e).

4. Method of anyone of claims 1 - 3, **characterized by** providing a resilient element that cooperates with the sleeve (28) in such a way, that the latter is axially sprung.

5. Method of anyone of claims 1 - 4, **characterized by** fixing the proximal end section (38) of the glass fibers (18) within the sleeve (28) by adhesive bonding.

6. Endoscope having a proximal light connection (64), the light connection (64) having a sleeve (28) in which a proximal end section (38) of glass fibers (18) held in the endoscope (18) is fixed for the purpose of light guidance, and in which the sleeve (28) is arranged in a connecting piece (22) of the light connection (64), wherein the sleeve (28) is designed in such a way that it can be introduced into the connecting piece (22) by a linear movement in the direction of its longitudinal axis, wherein a fiber cone (54) is arranged in a bearing fashion at an end surface (46) of the sleeve (28) and the glass fibers (18) contained therein, and wherein an optically conductive medium is introduced between the end surface (46) and the fiber cone (54).

7. Endoscope of claim 6, **characterized in that** the sleeve (28) is axially movable after being introduced into the connecting piece (22).

8. Endoscope of claim 7, **characterized in that** the sleeve (28) cooperates with a resilient element in such a way that it is axially sprung.

9. Endoscope of claim 8, **characterized in that** the resilient element is designed as O-ring (40).

10. Endoscope of anyone of claims 6 - 9, **characterized in that** the sleeve (28) is locked within the connecting piece (22).

11. Endoscope of anyone of claims 6 - 10, **characterized in that** the proximal end section (38) of the glass fibers (18) is fixed in the sleeve (28) by adhesive bonding.

## Revendications

1. Procédé pour fixer des fibres de verre (18) dans un endoscope (10), à un raccord de lumière proximal (64) de l'endoscope (10), lequel présente les étapes suivantes :
a) introduction d'une zone terminale proximale (38) des fibres de verre (18) dans un manchon (28) ;
b) introduction du manchon (28) dans une tubulure (22) du raccord de lumière (64) disposée sur l'endoscope (10) ;
c) fixation de la zone terminale proximale (38) des fibres de verre (18) dans le manchon (28) ;
d) séparation d'une partie terminale du manchon (28) avec les fibres de verre (18) qu'il contient ;
e) usinage d'une surface terminale (46) du manchon (28) et des fibres de verre (18) qu'il contient ; et
f) pose d'un cône de fibres (54) de façon que celui-ci soit appliqué contre la surface terminale,
un milieu optiquement conducteur étant introduit entre la surface terminale (46) et le cône de fibres (54) et l'introduction du manchon (28) dans la tubulure (22) étant réalisée par un déplacement linéaire dans la direction de son axe longitudinal.

2. Procédé selon la revendication 1, **caractérisé par** une introduction du manchon (28) en deux étapes, à savoir introduction dans une première position dans laquelle le manchon (28) est pressé dans la tubulure (22) et ensuite transfert dans une deuxième position, davantage enfoncée, dans laquelle le manchon (28) est verrouillé avec mobilité axiale dans la tubulure (22).

3. Procédé selon la revendication 2, **caractérisé par** un transfert du manchon (28) dans la deuxième position à la suite de l'étape e).

4. Procédé selon l'une des revendications 1 à 3, **caractérisé en ce qu'**il est prévu un élément élastique qui coopère avec le manchon (28) de sorte que celui-ci peut faire ressort en direction axiale.

5. Procédé selon l'une des revendications 1 à 4, **caractérisé par** une fixation de la zone terminale proximale (38) des fibres de verre (18) dans le manchon (28) par collage.

6. Endoscope, avec un raccord de lumière proximal (64), le raccord de lumière (64) présentant un manchon (28) dans lequel est fixée une zone terminale proximale (38) de fibres de verre (18) conductrices de lumière reçues dans l'endoscope (10), le manchon (28) étant disposé dans une tubulure (22) du raccord de lumière (64) et le manchon (28) étant conçu de façon à pouvoir être introduit dans la tubulure (22) par un mouvement linéaire dans la direction de son axe longitudinal, un cône de fibres (54) étant appliqué contre une surface terminale (46) du manchon (28) et des fibres de verre (18) qu'il contient, et un milieu optiquement conducteur étant introduit entre la surface terminale (46) et le cône de fibres (54).

7. Endoscope selon la revendication 6, **caractérisé en ce que** le manchon (28) est mobile axialement après l'introduction dans la tubulure (22).

8. Endoscope selon la revendication 7, **caractérisé en ce que** le manchon (28) coopère avec un élément élastique de manière à pouvoir faire ressort en direction axiale.

9. Endoscope selon la revendication 8, **caractérisé en ce que** l'élément élastique est réalisé sous la forme d'un joint torique (40).

10. Endoscope selon l'une des revendications 6 à 9, **caractérisé en ce que** le manchon (28) est verrouillé dans la tubulure (22).

11. Endoscope selon l'une des revendications 6 à 10, **caractérisé en ce que** la zone terminale proximale (38) des fibres de verre (18) est fixée dans le manchon (28) par collage.
